# EUROPEAN PATENT APPLICATION

(11) **EP 4 726 622 A1**
(43) Date of publication of application: **15.04.2026**
(21) Application number: 25206260.9
(22) Date of filing: 02.10.2025
(51) Int. Cl.: G06Q 10/0639, G06Q 50/08, A61B 5/16, A61B 5/18

(54) **WORK ASSISTANCE SYSTEM AND METHOD, NON-TRANSITORY STORAGE MEDIUM, AND SERVER DEVICE THEREFOR**

(30) Priority: 11.10.2024 JP 2024179375
(71) Applicant: Hiroshima University, Higashi-Hiroshima-shi Hiroshima 739-8511 (JP); KOBELCO CONSTRUCTION MACHINERY CO., LTD., Hiroshima-shi, Hiroshima 731-5161 (JP)
(72) Inventor: KINOSHITA, Takuya, Hiroshima-shi 739-8511 (JP); KOZUI, Masatoshi, Hiroshima-shi 731-5161 (JP); SAKAMOTO, Fumiya, Hiroshima-shi 731-5161 (JP)
(74) Representative: TBK

(57) **Abstract**

A work assistance system (1000) according to the present invention presents a working condition to an operator who performs a predetermined work, and acquires a psychological state of the operator, adjusts a working condition for the work on the basis of the acquired psychological state, and presents the operator with an adjusted working condition.

## Description

### Field of the Invention

The present invention relates to a work assistance system and a work assistance method for presenting a working condition to an operator who performs a predetermined work, a non-transitory storage medium storing a work assistance program, and a server device.

### Background Art

When performing a predetermined work, an operator performs the work on the basis of information presented thereto. For example, Japanese Unexamined Patent Publication No. 2016-076123 discloses a remote server that selects a work plan among a plurality of work plans on the basis of date and time information and work area, and sends the selected work plan to a terminal.

There is a likelihood that a working condition which is given to an operator to commence work affects a psychological state of the operator to generate an increased psychological burden to the operator. For example, when a working condition that would be perceived by operators as a difficulty is presented to an operator, the operator is liable to feel a psychological burden.

### Summary of the Invention

The present invention has been made in view of the circumstances described above, and an object thereof is to provide a work assistance system, a work assistance method, a non-transitory storage medium storing a work assistance program, and a server device that can suppress an increase in the psychological burden to the operator due to a working condition presentation, and consequently improve a psychological state of the operator.

A work assistance system and a work assistance method according to aspects of the invention acquire a psychological state of an operator who performs a predetermined work, adjust a working condition for the work on the basis of the acquired psychological state, and present an adjusted working condition to the operator. In a non-transitory storage medium storing a work assistance program according to another aspect of the invention, the work assistance program causes a computer to execute processing including acquiring a psychological state of an operator who performs a predetermined work, adjusting a working condition for the work on the basis of the acquired psychological state, and presenting an adjusted working condition to the operator. A server device according to still another aspect of the invention acquires a psychological state of an operator who performs a predetermined work, adjusts a working condition for the work on the basis of the acquired psychological state, and causes a presentation part for presenting the working condition to the operator to presents an adjusted working condition.

The foregoing and other objects, features, and advantages of the present invention will be further understood by the following detailed description with reference to accompanying drawings.

### Brief Description of the Drawings

FIG. 1 is a block diagram showing a configuration of a work assistance system according to an embodiment of the invention.
FIG. 2 is a diagram showing exemplary display images shown on a communication terminal device of the work assistance system.
FIG. 3 shows an exemplary working condition information table.
FIG. 4 shows an exemplary adjustment display image showing working conditions.
FIG. 5 is an exemplary graph showing a relationship between a value of a current psychological state of an operator and a target value.
FIG. 6 is an exemplary graph showing a readjustment of a working condition.
FIG. 7 is an exemplary graph showing a relationship between an elapsed time and a psychological state in a first manner.
FIG. 8 is an exemplary graph showing a relationship between an elapsed time and a psychological state in a second manner.
FIG. 9 is an exemplary graph showing a relationship between an elapsed time and a psychological state in a third manner.
FIG. 10 shows an exemplary lookup table to be used for the readjustment.
FIG. 11 is a flowchart showing an operation of the work assistance system.

### Description of Embodiments

Hereinafter, one or more embodiments of the present invention will be described with reference to the accompanying drawings. However, the present invention should not be limited to the disclosed embodiments. Elements denoted by the same reference numerals in the drawings have the same configuration and repeated descriptions will be appropriately omitted. In the present specification, an element is denoted by a reference numeral having no subscript when being referred to collectively, while an element is denoted by a reference numeral having a subscript when being referred to individually.

A work assistance system described in an embodiment presents a working condition to an operator who performs a predetermined work. The work assistance system includes an acquisition part, an adjustment part, and a presentation part. The acquisition part acquires a psychological state of an operator. The adjustment part adjusts a working condition for a work on the basis of the psychological state acquired by the acquisition part. The presentation part presents the operator with a working condition adjusted by the adjustment part. Hereinafter, the work assistance system is more specifically described together with a work assistance method, a work assistance program, and a server device that are in association with the system.

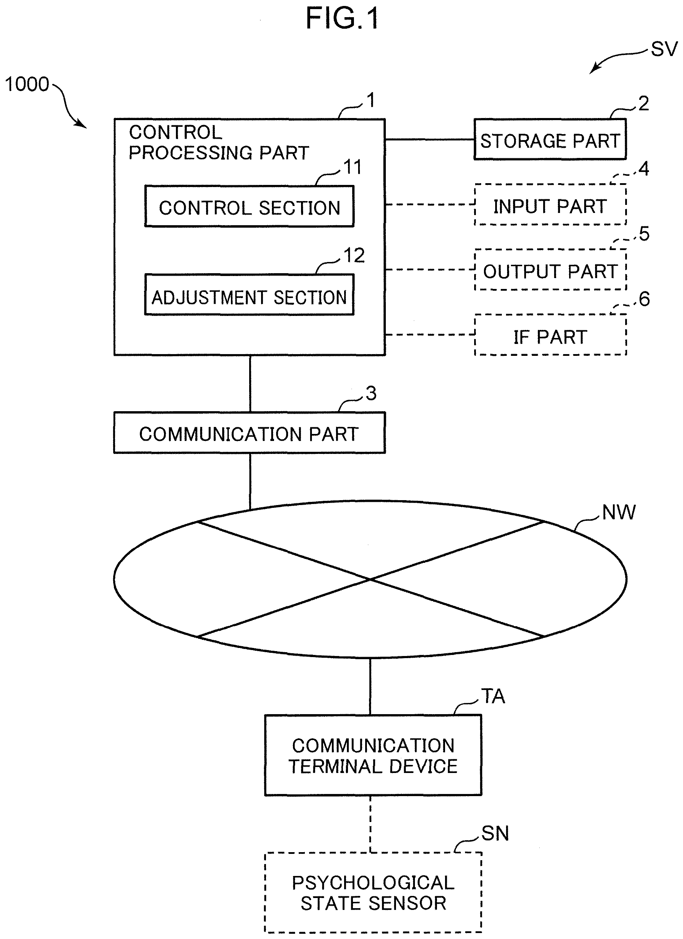

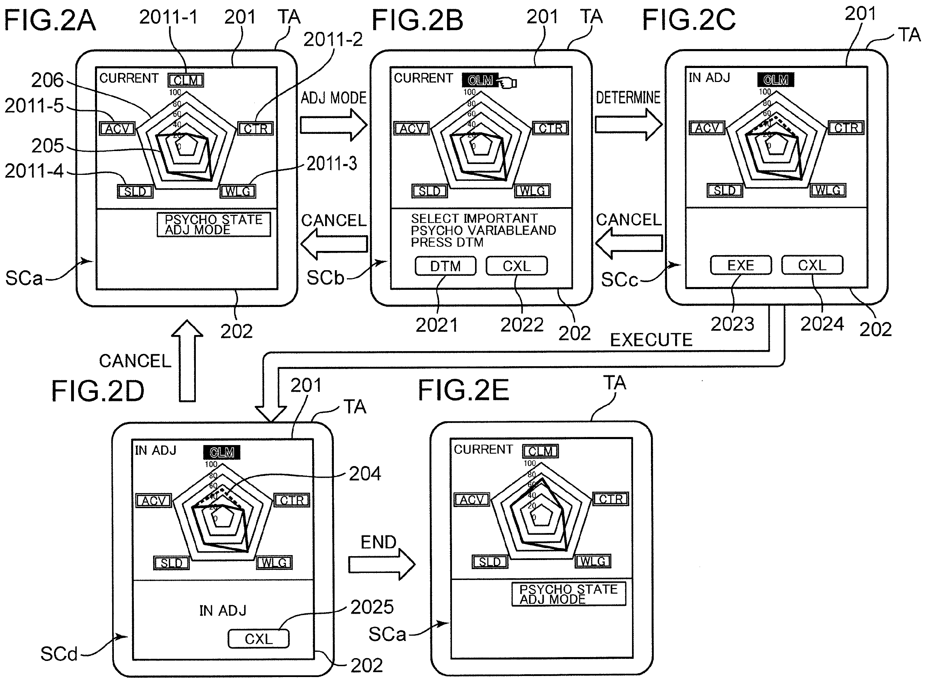

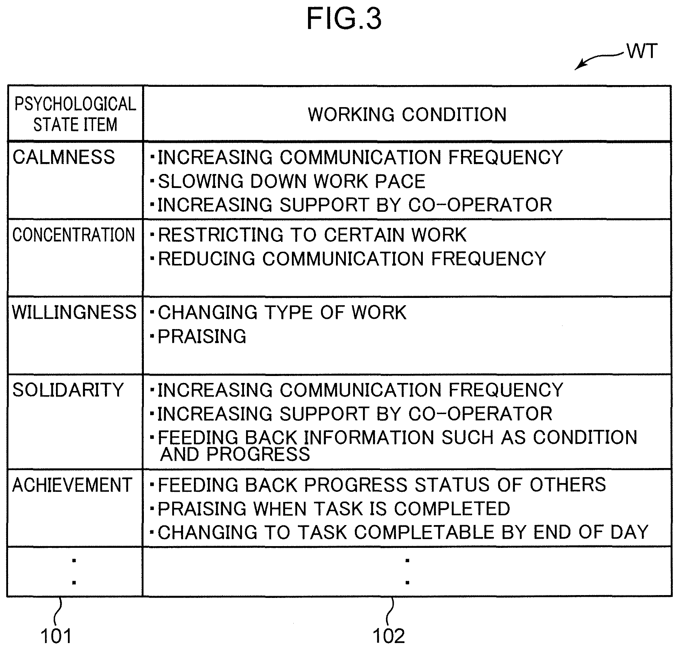

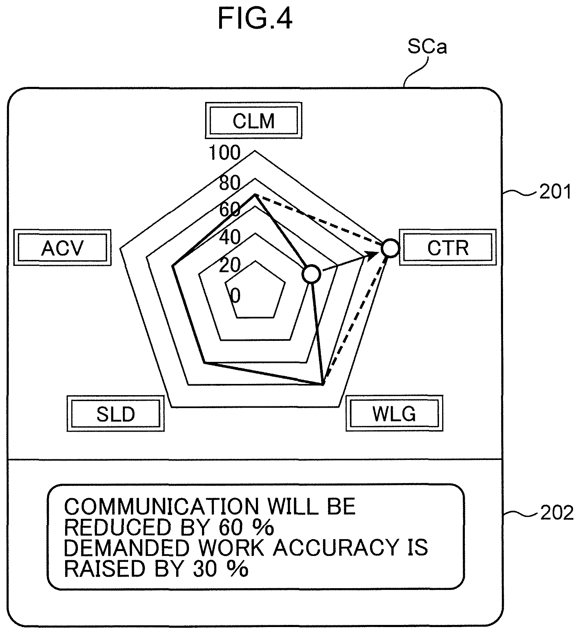

FIG. 1 is a block diagram showing a configuration of a work assistance system according to an embodiment. FIG. 2 is a diagram showing exemplary display images shown on a communication terminal device of the work assistance system. Each of FIG. 2A and FIG. 2E shows an exemplary display image (psychological state display image) that displays a current psychological state. FIG. 2B shows a display image (selection display image) in a selection of an item of the psychological state. FIG. 2C shows a display image (target display image) showing a target value of the psychological state for the selected item. FIG. 2D shows a display image (target execution image) for executing the setting of the target value of the psychological state. FIG. 3 shows an exemplary working condition information table. FIG. 4 shows an exemplary adjustment display image showing working conditions.

For example, a work assistance system 1000 of the embodiment presents a working condition to an operator who performs a predetermined work using a working machine, and includes a server device SV, a communication network NW, and a communication terminal device TA as shown in FIG. 1. The working machine is a machine operated by an operator to perform a predetermined work and includes, for example, a hydraulic excavator, a crane, and a wheel road roller.

The communication network NW includes a transmission channel, e.g., a telephone network, a digital communication network, and a wireless communication network. Data is transmitted via the communication network NW in accordance with a predetermined communication protocol. The communication network NW may be a WAN (Wide Area Network) or a LAN (Local Area Network).

The communication terminal device TA has a communication function of communicating with the server device SV via the communication network NW, an acquisition function of acquiring a psychological state of an operator in a working machine, and a presentation function of presenting a working condition to the operator for a work. For example, the communication terminal device TA includes a computer of laptop type or tablet type, and a so-called smartphone that have the communication function. For example, the communication terminal device TA is carried by an operator. Alternatively, for example, the communication terminal device TA is incorporated (implemented, mounted) in the working machine in such a manner that the operator can see and operate.

For example, the psychological state is represented in a degree with respect to each of a plurality of predetermined items. For example, the psychological state is represented in a degree with respect to each of Calmness, Concentration, Willingness, Solidarity, and Achievement. The items representing the psychological state are not limited to them but may include other items. For example, the psychological state is numerically represented in a percentage from 0 to 100 or in a 5-point Likert scale for each item, and is acquired by the communication terminal device TA that is input with a self-declared answer (an answer to a questionnaire) of an operator for each item.

Alternatively, for example, the psychological state may be measured and acquired by a psychological state sensor SN that measures predetermined items in connection with the psychological state, e.g., Calmness, Concentration, as shown by a dashed line in FIG. 1. The psychological state sensor SN includes well-known and commonly-used means such as a heart rate monitor and a blood pressure monitor that measure Calmness (Agitation), and an electroencephalograph that measures Concentration. The psychological state sensor SN is connected to the communication terminal device TA, and outputs an acquired result to the communication terminal device TA. The communication terminal device TA acquires a psychological state of the operator via the psychological state sensor SN.

Alternatively, for example, the psychological state may be estimated by the communication terminal device TA on the basis of an operation state of the working machine by the operator. Regarding the psychological state, a relationship between an operation state of the working machine and a degree with respect to each item of the psychological state is preliminarily defined as shown in Table 1, and a degree with respect to each item of the psychological state is estimated on the basis of Table 1. For example, a degree with respect to Willingness is estimated on the basis of whether an inoperative interval (an interval from an end time of a previous operation to a start time of a current operation) is long or short. An operation state detection sensor that detects an operation state of the working machine by the operator is connected to the communication terminal device TA. The inoperative interval is classified into three, i.e., Low (short), Mid (medium), and High (long) according to certain thresholds (classification thresholds). In a case where the inoperative interval based on a detection result by the operation state detection sensor is Low, Willingness is estimated to be high (e.g., 100 [%] or five in a five-point scale). In a case where the inoperative interval based on the detection result by the operation state detection sensor is Mid, Willingness is estimated to be medium (e.g., 50 [%] or three in the five-point scale). In a case where the inoperative interval based on the detection result by the operation state detection sensor is High, Willingness is estimated to be low (e.g., 0 [%] or one in the five-point scale).

**Table 1**

| Feature | Psychological State | | |
|---|---|---|---|
| | Low | Mid | High |
| Abrupt Operation Frequency | Agitation: Low (Good) | Agitation: Medium | Agitation: High (Bad) |
| Stop Accuracy | Concentration: Low (Bad) | Concentration: Medium | Concentration: High (Good) |
| Inoperative Interval | Willingness: High (Good) | Willingness: Medium | Willingness: Low (Bad) |
| Continuous Operation Time | Concentration: Low (Bad) | Concentration: Medium | Concentration: High (Good) |
| Wireless Response Time | Concentration: High (Good) | Concentration: Medium | Concentration: Low (Bad) |
| | Solitude: High (Bad) | Solitude: Medium | Solitude: Low (Good) |
| - | - | - | - |
| - | - | - | - |
| - | - | - | - |
| - | - | - | - |
| - | - | - | - |

An abrupt operation that is referred to hereinafter means an operation whose operational change per preset unit time is equal to or greater than a threshold (abrupt operation determination threshold). An abrupt operation frequency means a frequency of abrupt operation occurrence within a predetermined time. A degree of Agitation (Calmness) is estimated on the basis of the abrupt operation frequency. A stop accuracy means an adjustment amount to a stop position at which a work attachment is held, e.g., reverse adjustment of bringing an overrun work attachment in a reverse direction. The smaller the adjustment amount is, the higher the stop accuracy is. A degree of Concentration is estimated on the basis of the stop accuracy. A degree of Concentration is estimated on the basis of a continuous operation time. A degree of Concentration and a degree of Solitude (Solidarity) are estimated on the basis of a wireless response time. These estimations are based on preset relationships. For example, for the estimation of a degree of Agitation, a relationship between the abrupt operation frequency and the degree of Agitation is defined in advance, and this relationship is used.

The communication terminal device TA displays the acquired psychological state, displays a working condition for the work, and further receives a selection of an item among a plurality of items in connection with a psychological state, and receives a setting of the target value for the received item. The acquired psychological state and working condition are displayed on a display image of the communication terminal device TA shown in FIG. 2, for example. In FIG. 2A and FIG. 2E are shown psychological state display images SCa that display current psychological states. In FIG. 2B is shown a selection display image SCb that receives a selection of one or a plurality of items among the items in connection with a psychological state. In FIG. 2C is shown a target display image SCc that receives a setting of a target value of the psychological state for the selected item. In FIG. 2D is shown a target execution image SCd for executing the setting of the target value of the psychological state of the selected item.

Each of the psychological state display image SCa, the selection display image SCb, the target display image SCc, and the target execution image SCd includes a radar chart display region 201 where an acquired psychological state is shown in a radar chart and a working condition display region 202 where a working condition, a button, and the like are shown. The radar chart display region 201 and the working condition display region 202 are vertically arranged in the examples shown in FIG. 2. However, the radar chart display region 201 and the working condition display region 202 may be horizontally arranged or in different windows, for example. In the embodiment, the radar chart is provided with unillustrated numerical axes radially extending from a center of the radar chart and respectively corresponding to the items of Calmness, Concentration, Willingness, Solidarity, and Achievement. The respective values of Calmness, Concentration, Willingness, Solidarity, and Achievement in the acquired psychological state are plotted on the numerical axes corresponding to the respective items. A point plotted on a numerical axis and a point plotted on its adjacent numerical axis is connected by a line. A sequential connection of the adjacent points forms a polygonal chain 205. The radar chart has a value of zero at the center thereof, and values outwardly increasing from the center of the radar chart. The numerical axes are equivalently allotted with values from the center of the radar chart, and the value allotted points on a numerical axis and the value allotted points on its adjacent numerical axis are connected by lines. Sequential connections of the value allotted points form a plurality of regular-polygonal grids 206. In this embodiment, five regular-polygonal grids 206 are formed. The center of the radar chart is allotted with a value indicating a degree of 0 percent, and the five polygonal grids 206 are respectively allotted with values indicating degrees of 20 percent, 40 percent, 60 percent, 80 percent, and 100 percent in order from the innermost polygonal grid 206.

On the selection display image SCb, the radar chart shown in the radar chart display region 201 is provided with item indication fields 2011-1 to 2011-5 in respective extending directions of the numerical axes. The item indication fields 2011-1 to 2011-5 indicate item names of items of Calmness (CLM), Concentration (CTR), Willingness (WLG), Solidarity (SLD), and Achievement (ACV), respectively, and serve as input buttons for receiving the selection of an item to be set with a target value.

In the example shown in FIG. 2A, an item indicative of a psychological state adjustment mode (PSYCHO STATE ADJ MODE) is shown in the working condition display region 202 on the target display image SCa. The communication terminal device TA displays the image shown in FIG. 2B in response to a tap onto a screen of the communication terminal device TA or an operation to a button of the communication terminal device TA.

In the state where the communication terminal device TA displays the image shown in FIG. 2B, when an input operation is performed to a certain one of the item indication fields 2011-1 to 2011-5, the item of the item name indicated in the certain one of the item indication fields 2011-1 to 2011-5 is selected as an item to be set with a target value. The input operation is performed by placing a cursor on a certain one of the item indication fields 2011-1 to 2011-5 and pressing a return key in the case that the communication terminal device TA is a node computer. The input operation is performed by touching the certain one of the item indication fields 2011-1 to 2011-5 by a hand finger, a touch pen, or the like in the case that the communication terminal device TA is a tablet computer or a smartphone. In the example shown in FIG. 2B, the item indication field 2011-1 of Calmness is selected. When a plurality of items are selected, a plurality of item indication fields 2011-1 to 2011-5 are selected. A Determination (DTM) button 2021 for determining the selection of an item and a Cancelation (CXL) button 2022 for canceling the selection of an item are shown in the working condition display region 202 on the selection display image SCb. An input operation to the Determination button 2021 determines the item selected by the input operation to the one of the item indication fields 2011-1 to 2011-5 and inputs the item to the communication terminal device TA. An input operation to the Cancelation button 2022 cancels the selection of the item, making thus another item selection receivable. When the input operation to the Cancelation button 2022 is made by a long press, the communication terminal device TA returns to displaying the image shown in FIG. 2A.

In the state where the communication terminal device TA displays the image shown in FIG. 2B, an input operation to the Determination button 2021 on the selection display image SCb causes the communication terminal device TA to display the image shown in FIG. 2C, where the radar chart display region 201 receives setting of a target value. Performing an input operation to a point where the polygonal chain 205 intersects with the numerical axis of the item selected as the item to be set with a target value sets the target value. For example, a target value is set for each item by an input operation such as a swiping operation of moving a point of the polygonal chain 205 on a numerical axis to increase or decrease the value.

In the example shown in FIG. 2C, a Cancelation (CXL) button 2024 for canceling the set target value and an Execution (EXE) button 2023 for starting execution of a working condition adjustment for a work on the basis of the set target value are shown in the working condition display region 202 on the target display image SCc. The set target value is canceled, and the communication terminal device TA displays the image shown FIG. 2B in response to an input operation to the Cancelation button 2024. Thus, a target value can be set again. When an input operation to the Execution button 2023 is performed while the communication terminal device TA displays the image shown in FIG. 2C, a working condition adjustment for the work is started as shown in the target execution image SCd. When the working condition adjustment is started, the communication terminal device TA displays characters (a text) "in adjustment (IN ADJ)" and a Cancelation (CXL) button 2025 for canceling the adjustment in the working condition display region 202 on the target execution image SCd as shown in FIG. 2D. The adjustment of the psychological state is canceled and the display returns to a state before the start of the adjustment mode shown in FIG. 2A in response to an input operation to the Cancelation button 2025. When a predetermined time elapses from the start of the working condition adjustment, the working condition adjustment is completed, and the communication terminal device TA displays the image shown in FIG. 2E. In the example shown in FIG. 2E, characters (a text) "adjustment completed" may be displayed in the working condition display region 202 on the target display image SCc when the working condition adjustment is completed. In the example shown in FIG. 2E, the communication terminal device TA displays the image shown in FIG. 2B in response to a tap on the screen of the communication terminal device TA or an operation to a button provided in the communication terminal device TA. Thus, a working condition can be adjusted again.

In the examples shown in FIG. 2C and FIG. 2D, a target value for Calmness is set, and the set target value is connected to a value of Achievement in the acquired psychological state by a dashed line, and the set target value is connected to a value of Concentration in the acquired psychological state by a dashed line. When a plurality of items are selected, target values are respectively set for the items.

The communication terminal device TA displays the psychological state display image SCa, the selection display image SCb, and the target display image SCc by switching over them in response to an input operation.

The communication terminal device TA sends to the server device SV via the communication network NW psychological state information representing an acquired psychological state (in the example described above, the respective values of Calmness, Concentration, Willingness, Solidarity, and Achievement), target item information indicative of an item to be set with a target value, and target value information indicative of the target value.

The server device SV is communicably connected to the communication terminal device TA via the communication network NW to adjust a working condition for a work on the basis of a psychological state of an operator, and includes a control processing part 1, a storage part 2, and a communication part 3 as shown in FIG. 1, for example.

The communication part 3 is connected to the control processing part 1, and is communicably connected to the communication network NW. The communication part 3 generates a communication signal containing data that is inputted from the control processing part 1 and is to be sent (forwarded) in accordance with a communication protocol used by the communication network NW, and sends the generated communication signal to the communication terminal device TA via the communication network NW. The communication part 3 receives the communication signal from the communication terminal device TA via the communication network NW, extracts data from the received communication signal, converts the extracted data into a format in which the data can be processed by the control processing part 1, and outputs the data to the control processing part 1. For example, the communication part 3 is a communication interface circuit that sends and receives a communication signal to and from an external device, and includes a data communication card, and a communication interface circuit in conformity with IEEE 802. 11 standard.

The storage part 2 includes a circuit that is connected to the control processing part 1 and stores various predetermined programs and various kinds of predetermined data under the control by the control processing part 1.

The various predetermined programs include, for example, a control processing program. The control processing program includes a control program and an adjustment program, for example. The control program controls the parts 2, 3 of the server device SV according to functions of the respective parts. The adjustment program adjusts a working condition for a work on the basis of the psychological state acquired by the communication terminal device TA.

For example, the various kinds of predetermined data is data necessary to execute each program, and includes, for example, a working condition, a constraint, calculation results in the process of calculations and final calculation results. The storage part 2 includes, for example, a read only memory (ROM) that is a nonvolatile storage element, an electrically erasable programmable read only memory (EEPROM) that is a rewritable nonvolatile storage element, or the like. The storage part 2 includes a random access memory (RAM) serving as a so-called working memory of the control processing part 1 that stores, e.g., data generated in an execution of predetermined programs. The storage part 2 may include a hard disk device and a solid state drive (SSD) that has a relatively large capacity.

The working condition includes a predetermined condition imposed to an operator in performing a work, a working environment around the operator, a relationship with a co-operator, and is defined in advance for each item of the psychological state and stored in the storage part 2 in a table format. The working condition preferably includes a numerical value and a level that determine the working condition to make the working condition adjustable, and preferably includes in a plurality of kinds. For example, as shown in FIG. 3, a working condition information table WT for recording the working conditions includes psychological state item fields 101 for recording the respective item names of the items of the psychological state and working condition fields 102 for recording working conditions for each of the items having the item names recorded in the psychological state item fields 101. The working condition information table WT contains a record for each item of the psychological state. In the example shown in FIG. 3, a working condition corresponding to Calmness is a condition for calming down, i.e., increasing the calmness, and more specifically includes "increasing a communication or conversation frequency by a predetermined value X₁₁ [%]", "slowing down a work pace by a predetermined value X₁₂ [%], i.e., lowering the productivity by the predetermined value X₁₂ [%]", and "increasing operator support, i.e., assistance, help, by a certain number X₁₃ of persons." A working condition corresponding to Concentration is a condition for improving a low concentration, and more specifically includes "restricting to a certain type X₂₁ of work", and "reducing the communication or conversation frequency by a predetermined value X₂₂ [%]." A working condition corresponding to Willingness is a condition for increasing the willingness, i.e., raising the willingness, and specifically includes "changing the type of work to a predetermined type X₃₁ of work", "praising at a certain level X₃₂, e.g., praising exaggeratedly, praising moderately, praising modestly". The working condition corresponding to Concentration may include a condition for lowering an excessively high concentration, more specifically, "removing a restriction to the certain type X₂₁ of work" or "increasing the communication or conversation frequency by a predetermined value X₂₃ [%]". A working condition corresponding to Solidarity is a condition for raising a low solidarity with a co-operator, and more specifically includes "increasing the communication or conversation frequency by a predetermined value X₄₁ [%]", "increasing the operator support, i.e., assistance, help, by a predetermined number X₄₂ of persons", or "feeding back information such as condition and progress (increasing a feedback frequency by a certain value X₄₃)". A working condition corresponding to Achievement is a condition for raising sense of achievement, and more specifically includes "feeding back a progress status of another operator (increasing the feedback frequency by a certain value X₅₁)", "praising at a certain level X₅₂ when a task is completed", or "changing to a task having a certain amount X₅₃ completable by the end of the day".

The constraint is a condition specifying a range (adjustable range) within which the adjustment can be performed when a working condition is adjusted. For example, in a case that the constraint is the number of operators available to support and the sense of solidarity is raised by increasing the operator support, the number of operators available to support is given an upper limit, and the working condition cannot be adjusted to the number of supporting operators greater than the upper limit. For example, in a case that the constraint is a weather type such as a thunderstorm and a strong wind and the willingness is increased by changing the type of work, the working condition cannot be adjusted by changing to a type of work which is not performable under a specified weather type. For example, in the case of rain, a work of forming a slope, i.e., slope formation work, cannot be performed. Thus, the type of work cannot be changed to the slope formation work. For example, in a case that the constraint is progress and the operator is calmed down by slowing down the work pace, i.e., lowering the productivity, the work pace is given a lower limit on the basis of a current progress. The working condition cannot be adjusted to slow down the work pace beyond the lower limit. Also, when the current progress is behind a progress defined in a work plan by a certain degree or more, the work pace cannot be slowed down. Further, for example, in a case that the constraint is the number (i.e., vehicle number) of transport vehicles such as dump trucks and the operator is calmed down by slowing down the work pace (i.e., lowering the productivity), the work pace is given a lower limit on the basis of the number of transport vehicles. The working condition cannot be adjusted to slow down the work pace beyond the lower limit.

The work assistance program may be either recorded in a non-transitory recording medium and stored in the storage part 2 or downloaded via a network and stored in the storage part 2.

The control processing part 1 includes a circuit that controls the parts 2, 3 of the server device SV in the work assistance system 1000 according to functions of the respective parts to thereby adjust the working condition for a work on the basis of the psychological state acquired from the communication terminal device TA. For example, the control processing part 1 includes a central processing unit (CPU) and a peripheral circuitry thereof. The execution of the control processing program causes the control processing part 1 to functionally include a control section 11 and an adjustment section 12.

The control section 11 administers the entire control of the server device SV by controlling the parts 2, 3 of the server device SV in the work assistance system 1000 according to functions of the respective parts. The control section 11 controls the communication part 3 to receive from the communication terminal device TA via the communication network NW a communication signal (psychological state etc. notification signal) including the psychological state information representing the psychological state acquired by the communication terminal device TA, the target item information indicative of an item to be set with a target value, and the target value information indicative of the target value. The control section 11 generates a communication signal (i.e., working condition notification signal) including working condition information representing a working condition adjusted by the adjustment section 12 as described below and controls the communication part 3 to send the generated working condition notification signal to the communication terminal device TA via the communication network NW in order to cause the communication terminal device TA to present the operator with the working condition adjusted by the adjustment section 12.

The adjustment section 12 adjusts the working condition for a work on the basis of the psychological state acquired by the communication terminal device TA. The working condition includes a condition regarding the productivity of the work. The adjustment section 12 adjusts, in order to suppress an increase in the psychological burden to the operator, the productivity of the working condition so as to lower or raise the productivity demanded before the adjustment of the working condition. The working condition includes a condition regarding an instruction given to the operator, and the adjustment section 12 adjusts, in order to suppress an increase in the psychological burden to the operator, the content or level of the instruction of the working condition so as to lower or raise the content or level of the instruction before the adjustment of the working condition. For example, the instruction includes communication or conversation. The instruction is information to be recognized or known by the operator and a working condition proposed to the operator when performing a work, and includes, for example, a guidance on a work procedure, a guidance on cautions in the work, a guidance on matters to be kept in mind when performing the work, a guidance on how to operate a working machine, and a work progress status. The content or level of the instruction is adjusted by decreasing (i.e., reducing or lowering) the communication frequency.

More specifically, the adjustment section 12 adjusts the working condition by obtaining a numerical value, level, type, and the like of the working condition on the basis of a difference between the current psychological state acquired by the communication terminal device TA and the target value. In a case that the operator is calmed down by slowing down the work pace, i.e., lowering the productivity, for example, when the current Calmness is 40 and the target value is 100, the work pace is lowered by 50 percent since the difference is 60. When the current Calmness is 40 and the target value is 80, the work pace is lowered by 20 percent since the difference is 40. In a case that the Concentration is increased by decreasing the communication frequency, for example, when the current Concentration is 40 and the target value is 100, the communication frequency is decreased by 60 percent since the difference is 60. When the current Concentration is 40 and the target value is 80, the communication frequency is decreased by 30 percent since the difference is 40. A relationship between the difference and the value, level, type, and the like of the working condition is properly defined in advance. A plurality of working conditions may be adjusted for a single item of the psychological state.

When the working condition is adjusted by the adjustment section 12, as described above, the control section 11 generates the working condition notification signal and causes the communication part 3 to send the generated working condition notification signal to the communication terminal device TA via the communication network NW in order to cause the communication terminal device TA to present the operator with the working condition adjusted by the adjustment section 12.

When receiving the working condition notification signal, the communication terminal device TA extracts the working condition information from the received working condition notification signal, displays the working condition shown in the working condition information, presenting thus the working condition to the operator. FIG. 4 shows an example of the presentation. In the example shown in FIG. 4, a message "Communication will be reduced by 60 percent" and a message "Demanded work accuracy is raised by 30 percent" are shown in the working condition display region 202 in order to raise the Concentration to the target value of 100.

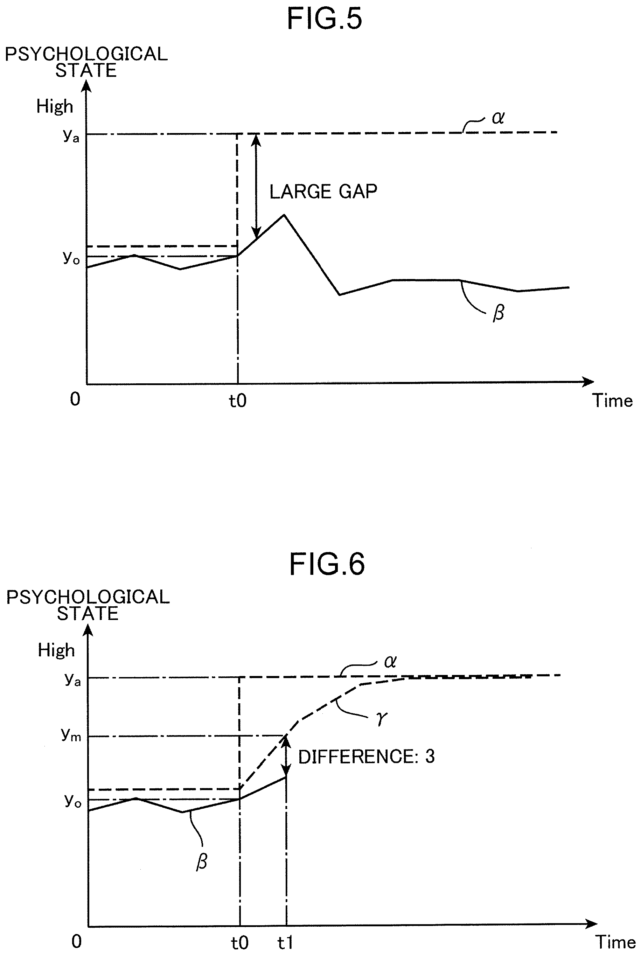

As described above, the communication terminal device TA may present the working condition adjusted by the adjustment section 12. However, as shown in FIG. 5, when the working condition is adjusted so that the psychological state reaches a target psychological state, there is a likelihood that the difference or gap between the current working condition and the adjusted working condition increases and the presentation of the adjusted working condition rather increases the psychological burden to the operator. Therefore, in this embodiment, the adjustment section 12 obtains a sub-target psychological state at a time in a predetermined elapse from the current time on the basis of a relationship between a time elapse and a psychological state in a time course of approaching the target psychological state, and readjusts the adjusted working condition on the basis of the obtained sub-target psychological state, and the communication terminal device TA presents the operator with a working condition readjusted by the adjustment section 12.

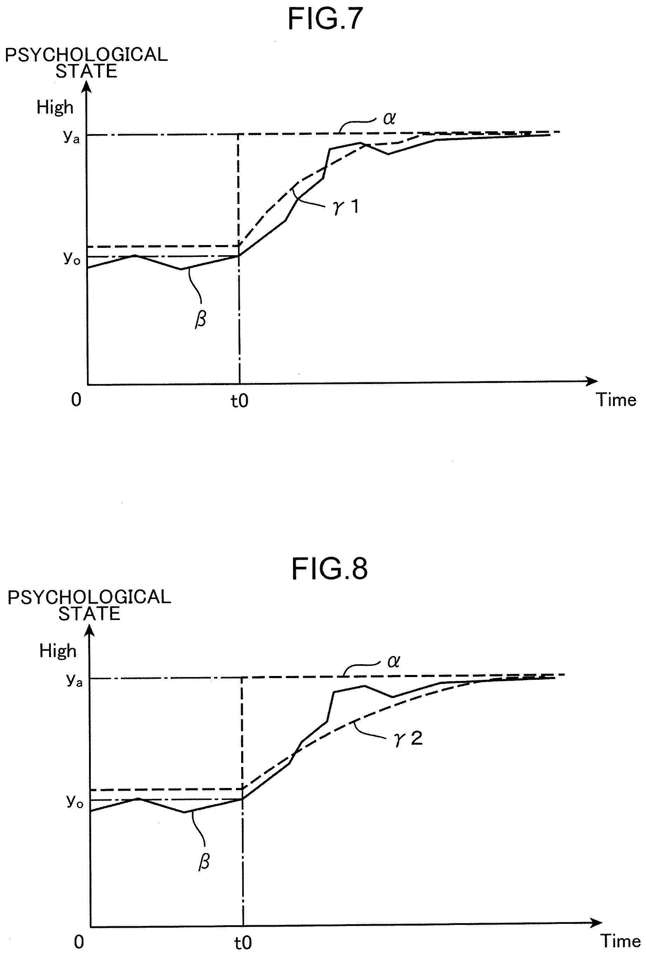

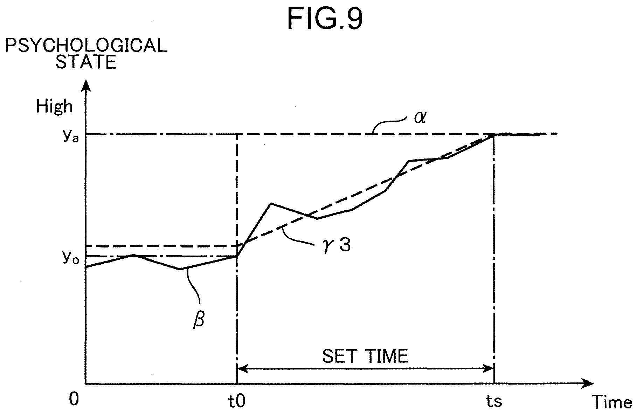

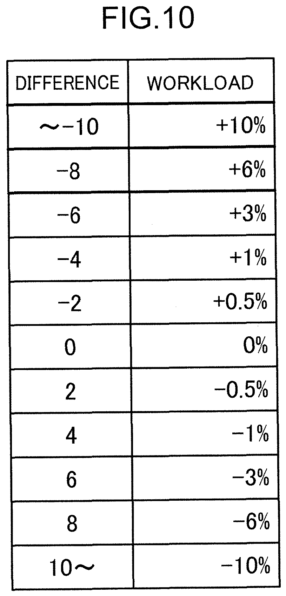

FIG. 5 is an exemplary graph showing a relationship between a value of a current psychological state of an operator and a target value. FIG. 6 is an exemplary graph showing a readjustment of a working condition. FIG. 7 is an exemplary graph showing a relationship between an elapsed time and a psychological state in a first manner. FIG. 8 is an exemplary graph showing a relationship between an elapsed time and a psychological state in a second manner. FIG. 9 is an exemplary graph showing a relationship between an elapsed time and a psychological state in a third manner. In FIG. 5 to FIG. 9, each horizontal axis indicates time, and each vertical axis indicates a psychological state. Graph α represents a target value of a psychological state. Graph β represents a psychological state of the operator. Graph γ represents a relationship between a time elapse and a psychological state in the time course of approaching the target psychological state. Time t0 indicates a current time. Time t1 indicates a time in a predetermined elapse from the current time. Psychological state y0 indicates a psychological state of the operator at the current time t0, and psychological state ya indicates a target value of the psychological state. FIG. 10 shows an exemplary lookup table to be used for readjustment.

More specifically, the adjustment section 12 first obtains a sub-target psychological state (a sub-target value) that is the psychological state ym at the time t1 in the predetermined elapse from the current time t0 on the basis of the relationship γ between the time elapse and the psychological state that approaches the target psychological state ya in the time course.

The relationship γ is properly defined in advance. For example, the relationship γ includes the following relationships γ1 to γ3 in the first to third manners.

For example, as shown in FIG. 7, the relationship γ1 in the first manner is a transfer function K/ (1 + Ts) of a first-order lag, that is, γ1(s) = K/ (1 + Ts), wherein K denotes a gain, T denotes a time constant, and s denotes a Laplace operator. The relationship γ1 in the first manner is expressed in the time domain as y(t) = (K/T) × e ^{(-t/T)}, wherein e denotes Napier's constant.

For example, the relationship γ2 in the second manner is a relationship in accordance with a so-called Weber's law (i.e., Weber-Fechner law) as shown in FIG. 8. According to Weber's law, a ratio ΔC/C of a psychological state variation ΔC to the current psychological state C is constant (ΔC/C = a constant).

The relationship γ3 in the third manner is a so-called ramp function as shown in FIG. 9, for example. When a time until reaching the target value from the current time t0 is denoted by ts, y(t) = y0 + ((ya - y0) / (ts - t0)) × t. The time ts is properly set in advance.

Next, the adjustment section 12 readjusts the adjusted working condition on the basis of the obtained sub-target psychological state ym. For example, in the example described above, when the operator is calmed down by slowing down the work pace (or lowering the productivity) and the current Calmness (at the current time t0) is 40 and the target value is 100, the work pace is lowered by 50 percent since the difference is 60. When the sub-target value ym is 55, i.e., the current Calmness (at the current time t0) is 40 and the sub-target value is 55, the work pace is lowered by 10 percent since the difference is 15, and the working condition is readjusted. A relationship between the difference and the numerical value, level, type and the like of the working condition in the readjustment is properly defined in advance. For example, a lookup table shown in FIG. 10 is prepared in advance and stored in the storage part 2. For example, a percentage shown in FIG. 10 is added to or subtracted from the current workload (demanded production amount) on the basis of a difference between the current value and the sub-target value. For example, when the difference (= sub-target value - current value) is 11 and the demanded production amount is 5, the corrected demanded production amount is obtained as 5 × (1 - 0.1) = 4.5. When the difference is -6 and the demanded production amount is 4, the corrected demanded production amount is obtained as 4 × (1 + 0.03) = 4.12. The demanded production amount is sequentially corrected in this way. Alternatively, for example, a readjusted working condition is obtained by a feedback control in a PID control based on a difference Δy between the psychological state y0 at the current time t0 and the sub-target value ym. For example, when the operator is calmed down by slowing down the work pace by the predetermined value X₁₂ [%] (or lowering the productivity by the predetermined value X₁₂ [%]), the predetermined value X₁₂ is obtained by X₁₂ = Kp × Δy(t) + Ki × ∫y(τ)dτ + Kd (dΔy(t) / dt), wherein Kp denotes a proportional gain, Ki denotes an integral gain, Kd denotes a derivative gain, and an integral ∫ is an integral from 0 to t.

When the working condition is readjusted by the adjustment section 12, the control section 11 generates a working condition notification signal containing working condition information representing a working condition readjusted by the adjustment section 12 instead of the above-described generation of the working condition notification signal including the working condition information representing the working condition adjusted by the adjustment section 12, and sends the generated working condition notification signal to the communication terminal device TA via the communication network NW in order to cause the communication terminal device TA to present the operator with the working condition readjusted by the adjustment section 12.

When receiving the working condition notification signal, the communication terminal device TA extracts the working condition information from the received working condition notification signal and displays the working condition (the readjusted working condition) shown in the working condition information, presenting thus the working condition to the operator.

As shown by the dotted lines in FIG. 1, the server device SV may further include an input part 4 that is connected to the control processing part 1 and inputs various commands and various data to the server device SV, an output part 5 that is connected to the control processing part 1 and outputs the commands and the data that are input from the input part 4, calculation results, and the like under the control by the control processing part 1, and an interface part (IF part) 6 that is connected to the control processing part 1 and inputs and outputs data to and from an external device under the control by the control processing part 1.

For example, the server device SV in the embodiment may be constituted by a computer of desktop type, tower type, or the like.

The readjustment of the working condition is performed by the server device SV in the above description. However, the readjustment may be performed by the communication terminal device TA.

In the above description, the work assistance system 1000 includes the server device SV and the communication terminal device TA. However, the work assistance system 1000 may be constituted by: an input/output terminal device that inputs and outputs data; one or a plurality of processors (e.g., server devices) that performs various operations; and one or a plurality of data base devices that stores (manages) various data, that are communicably connected with one another. Alternatively, some of the input/output terminal device, the one or plurality of processors, and the one or plurality of data base devices may be integrated to one body and communicably connected with the remaining parts, or all the parts may be integrated to one body.

In the above description, the communication terminal device TA receives selection of an item to be set with a target value among the plurality of items of the psychological state, and receives the setting of the target value for the received item. However, an input/output terminal device provided separately from the communication terminal device TA may receive the selection of the item to be set with a target value and receive the setting of the target value for the received item. In this configuration, the communication terminal device TA acquires a psychological state of an operator of a working machine, generates a communication signal (psychological state notification signal) including psychological state information representing the acquired psychological state of the operator and sends the psychological state notification signal to the server device SV, and the input/output terminal device generates a communication signal (target notification signal) including the target item information indicative of an item to be set with a target value, and the target value information indicative of the target value, and sends the target notification signal to the server device SV. Since the work assistance system 1000 includes the input/output terminal device provided separately from the communication terminal device TA, the work assistance system 1000 enables a manager such as a site director who manages a construction site or a worksite to select an item to be set with a target value and set the target value. Alternatively, the server device SV may receive the selection of an item to be set with a target value and receive the setting of the target value for the received item. In this configuration, the communication terminal device TA acquires a psychological state of an operator of the working machine, generates a psychological state notification signal, and sends the psychological state notification signal to the server device SV. In a configuration including a psychological state sensor SN, the psychological state sensor SN may directly send the psychological state of the operator to the server device SV in place of the communication terminal device TA. This configuration also enables a manager to select an item to be set with a target value and set the target value.

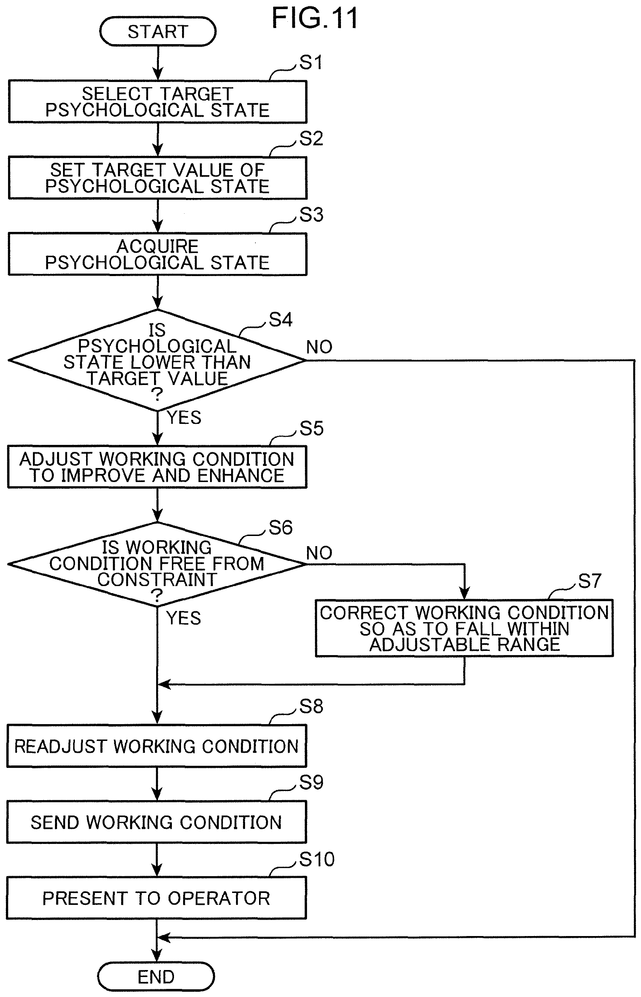

Next, an operation in the embodiment is described. FIG. 11 is a flowchart showing an operation of the work assistance system.

The server device SV configured as described above initializes necessary parts, and starts the operation when a power source thereof is turned on. The execution of the control processing program causes the control processing part 1 to functionally include the control section 11 and the adjustment section 12. The communication terminal device TA is preliminarily installed with an application software for implementing an acquisition function of acquiring a psychological state of an operator of a working machine, an adjustment function of adjusting a working condition for a work on the basis of the psychological state acquired using the acquisition function, and a presentation function of presenting the operator with the working condition for the work.

In FIG. 11, the communication terminal device TA acquires a psychological state of an operator of a working machine, and receives an input operation of a certain item to be set with a target value among the item indication fields 2011-1 to 2011-5 while displaying a polygonal chain 205 corresponding to the acquired psychological state of the operator together with the five polygonal grids 206 and the respective item names in the indication fields 2011-1 to 2011-5, thus recognizing that the certain item of the item name indicated in one of the item indication fields 2011-1 to 2011-5 that has received the input operation is selected as the item of the psychological state to be set with a target value (S1).

Further, the communication terminal device TA receives an input operation of shifting a point where the polygonal chain 205 intersects with the numerical axis of the certain item of the psychological state selected in Step S1 to the point of the target value, thus recognizing that the target value of the psychological state is set (S2).

Further, the communication terminal device TA generates a psychological state etc. notification signal including the psychological state of the operator of the working machine acquired in Step S1, the certain item of the psychological state recognized in Step S1, and the target value of the psychological state recognized in Step S2, and sends the generated psychological state etc. notification signal to the server device SV via the communication network NW, causing thus the server device SV to acquire the psychological state etc. notification signal (S3).

Further, when receiving the psychological state etc. notification signal by the communication part 3, the server device SV extracts psychological state information representing a psychological state of the operator, target item information indicative of an item of the psychological state of the operator, and target value information indicative of a target value of the psychological state of the operator from the received psychological state etc. notification signal by the control section 11 of the control processing part 1, and determines whether the psychological state of the certain item to be set with the target value is lower than the target value by the adjustment section 12 of the control processing part 1 (S4). When the psychological state is not lower than the target value (No) as a result of this determination (when the psychological state is equal to or higher than the target value), the server device SV ends the process. On the other hand, when the psychological state is lower than the target value (Yes) as a result of this determination, the server device SV subsequently executes a proceeding in Step S5.

In the proceeding in Step S5, the server device SV adjusts at the adjustment section 12 a working condition for a work on the basis of a psychological state of the item to be set with the target value.

Further, the server device SV determines at the adjustment section 12 whether the working condition is free from constraint (S6). When the working condition is not free from constraint (No) as a result of the determination, the server device SV subsequently executes a proceeding in Step S7, and then a proceeding in Step S8. On the other hand, when the working condition is free from constraint (Yes) as a result of the determination, the server device SV subsequently executes the proceeding in Step S8.

In Step S7, the server device SV corrects at the adjustment section 12 the working condition adjusted in Step S5 so as to meet the constraint. In other words, the adjustment section 12 corrects the working condition adjusted in Step S5 in such a manner as to fall within an adjustable range specified by the constraint.

In Step S8, the server device SV readjusts at the adjustment section 12 the adjusted working condition.

Further, the server device SV generates at the adjustment section 12 working condition information showing the readjusted working condition, generates at the control section 11 a working condition notification signal containing the working condition information, and sends the working condition notification signal to the communication terminal device TA (S9) by the communication part 3.

When receiving the working condition notification signal, the communication terminal device TA extracts working condition information from the received working condition notification signal, displays the working condition shown in the extracted working condition information to thereby present the working condition to the operator (S10), and ends the process.

The proceedings in Steps S1 to S10 are repeated every predetermined time. In the repetition, Steps S2 and S3 may be omitted to retain the initial certain item to be set with a target value and the initial target value.

As described above, in the work assistance system 1000, and the work assistance method and the work assistance program that are in association with the system, a working condition for a work is adjusted on the basis of a psychological state and the adjusted working condition is presented. Thus, the system, method and program can suppress an increase in the psychological burden to an operator due to a working condition presentation, and consequently improve a psychological state of the operator. For example, a positive emotion such as a motivation of the operator for the work can be kept from lowering.

The work assistance system 1000, the work assistance method, and the work assistance program adjust, in order to suppress an increase in the psychological burden to an operator, the productivity so as to lower or raise the productivity demanded before the adjustment of the working condition. Therefore, the system, method and program can suppress an increase in the psychological burden to the operator due to the presentation of an excessively high productivity for the operator and keep the psychological state from worsening due to the presentation of an excessively low productivity for the operator, improving thus the psychological state of the operator.

The work assistance system 1000, the work assistance method, and the work assistance program adjust, in order to suppress an increase in the psychological burden to the operator, a content or level of an instruction so as to lower or raise the content or level of the instruction before the adjustment. Therefore, the system, method and program can suppress an increase in the psychological burden to the operator due to the presentation of an excessively large amount of instruction for the operator or the presentation of an excessively small amount of instruction for the operator.

The work assistance system 1000, the work assistance method, and the work assistance program readjust the adjusted working condition on the basis of a sub-target psychological state and presents the readjusted working condition. Therefore, the system, method and program can lead the operator to a target psychological state while suppressing an increase in the psychological burden to the operator, and gradually lead the operator to a state in which the operator can perform a work more commensurate with the working condition.

The work assistance system 1000, the work assistance method, and the work assistance program present a working condition to an operator who performs a predetermined work using a working machine. However, the system, method and program are not limited to them, but may present the working condition to an operator who performs a predetermined work in a construction site or a work site without using any working machine. For example, at least a part of a work of carrying, loading and discharging a material used in a construction site or a work site, a demolishing work, an electrical wiring work, a piping installation work, a painting work, a refining work, a cleaning work is the work performed without using any working machine. The system, method, and program may present a working condition to an operator who performs one of them.

The present specification has disclosed technologies having various aspects as described above, and main technologies are summarized hereinafter.

A work assistance system according to an aspect presents a working condition to an operator who performs a predetermined work, and includes an acquisition part that acquires a psychological state of the operator, an adjustment part that adjusts a working condition for the work on the basis of the psychological state acquired by the acquisition part, and a presentation part that presents the operator with a working condition adjusted by the adjustment part.

The work assistance system adjusts the working condition for a work on the basis of a psychological state and presents the adjusted working condition. Therefore, the system can suppress an increase in the psychological burden to the operator due to a working condition presentation, improving thus the psychological state of the operator.

In another configuration, it may be appreciated that in the work assistance system, the working condition includes a condition regarding a productivity of the work, and the adjustment part adjusts, in order to suppress an increase in the psychological burden to the operator, the productivity of the working condition so as to lower or raise the productivity demanded before the adjustment of the working condition.

The work assistance system adjusts the productivity so as to lower or raise the productivity before the adjustment. Therefore, the work assistance system can suppress an increase in the psychological burden to the operator due to a productivity presentation, improving thus the psychological state of the operator.

In another configuration, it may be appreciated that in the work assistance system described above, the working condition includes a condition regarding an instruction given to the operator, and the adjustment part adjusts, in order to suppress an increase in the psychological burden to the operator, the content or level of the instruction of the working condition so as to lower or raise the content or level of the instruction before the adjustment of the working condition.

The work assistance system adjusts the content or level of an instruction so as to lower or raise a content or level of the instruction before the adjustment. Therefore, the work assistance system can suppress an increase in the psychological burden to the operator due to an instruction presentation, improving thus the psychological state of the operator.

In still another configuration, in the work assistance system described above, it may be appreciated that the adjustment part obtains a sub-target psychological state at a time in a predetermined elapse from a current time on the basis of a relationship between a time elapse and a psychological state in a time course of approaching to a target psychological state, and readjusts the adjusted working condition on the basis of the obtained sub-target psychological state, and the presentation part presents the operator with a working condition readjusted by the adjustment part.

When the working condition is adjusted so that the psychological state reaches a target psychological state, there is a likelihood that the difference or gap between the current working condition and the adjusted working condition increases and the presentation of the adjusted working condition rather increases the psychological burden to the operator. The work assistance system readjusts the adjusted working condition on the basis of the sub-target psychological state and presents the readjusted working condition. Therefore, the system can lead the operator to the target psychological state while suppressing an increase in the psychological burden to the operator.

In still another configuration, in the work assistance system described above, the work may be performed by the operator using a working machine.

This configuration is embodied as the work assistance system for a work performed by the operator using a working machine.

A server device according to another aspect presents a working condition to an operator who performs a predetermined work, and includes an acquisition part that acquires a psychological state of the operator, an adjustment part that adjusts the working condition for the work on the basis of the psychological state acquired by the acquisition part, and a control processing part that causes a presentation part for presenting the working condition to the operator to present a working condition adjusted by the adjustment part.

The server device adjusts a working condition for a work on the basis of a psychological state and presents the adjusted working condition. Therefore, the server device can suppress an increase in a psychological burden to the operator due to a working condition presentation, improving thus the psychological state of the operator.

A work assistance method according to still another aspect presents a working condition to an operator who performs a predetermined work, and includes an acquisition step of acquiring a psychological state of an operator, an adjustment step of adjusting the working condition for the work on the basis of the psychological state acquired in the acquisition step, and a presentation step of presenting the operator with a working condition adjusted in the adjustment step.

The work assistance method adjusts a working condition for a work on the basis of a psychological state and presents the adjusted working condition. Therefore, the work assistance method can suppress an increase in the psychological burden to the operator due to a working condition presentation, improving thus the psychological state of the operator.

A non-transitory storage medium according to still another aspect records a work assistance program causing a computer to execute an acquisition process of acquiring a psychological state of an operator who performs a predetermined work, an adjustment process of adjusting a working condition for the work on the basis of the psychological state acquired in the acquisition process, and a presentation process of causing a presentation part for presenting the working condition to the operator to present a working condition adjusted in the adjustment process.

The working assistance program recorded in the non-transitory storage medium adjusts a working condition for a work on the basis of a psychological state and presents the adjusted working condition. Therefore, the program can suppress an increase in the psychological burden to the operator due to a working condition presentation, improving thus the psychological state of the operator.

This application is based on Japanese Patent Application No. 2024-179375 filed on October 11, 2024, the contents of which are incorporated in the present application.

Although the present invention has been fully described by way of example with reference to the accompanying drawings, it is to be understood that various changes and modifications will be apparent to those skilled in the art. Therefore, unless otherwise such changes and modifications depart from the scope of the present invention hereinafter defined, they should be construed as being included therein.

## Claims

1. A work assistance system (1000) for presenting a working condition to an operator who performs a predetermined work, comprising:
an acquisition part (3, TA, SN) that acquires a psychological state of an operator;
an adjustment part (12) that adjusts a working condition for a work on the basis of the psychological state acquired by the acquisition part; and
a presentation part (TA) that presents the operator with a working condition adjusted by the adjustment part.

2. The work assistance system according to claim 1, wherein
the working condition includes a condition regarding a productivity of the work, and the adjustment part adjusts, in order to suppress an increase in the psychological burden to the operator, the productivity of the working condition so as to lower or raise the productivity demanded before the adjustment of the working condition.

3. The work assistance system according to claim 1 or 2, wherein
the working condition includes a condition regarding an instruction given to the operator, and
the adjustment part adjusts, in order to suppress an increase in the psychological burden to the operator, the content or level of the instruction of the working condition so as to lower or raise the content or level of the instruction before the adjustment of the working condition.

4. The work assistance system according to any one of claims 1 to 3, wherein
the adjustment part obtains a sub-target psychological state at a time in a predetermined elapse from a current time on the basis of a relationship between a time elapse and a psychological state in a time course of approaching to a target psychological state, and readjusts the adjusted working condition on the basis of the obtained sub-target psychological state, and
the presentation part presents the operator with a working condition readjusted by the adjustment part.

5. The work assistance system according to any one of claims 1 to 4, wherein
the work is performed by the operator using a working machine.

6. A server device (SV) for presenting a working condition to an operator who performs a predetermined work, comprising:
an acquisition part (3) that acquires a psychological state of an operator;
an adjustment part (12) that adjusts the working condition for the work on the basis of the psychological state acquired by the acquisition part; and
a control processing part (1) that causes a presentation part (TA) for presenting the working condition to the operator to present a working condition adjusted by the adjustment part.

7. A work assistance method for presenting a working condition to an operator who performs a predetermined work, comprising:
an acquisition step (S3) of acquiring a psychological state of an operator;
an adjustment step (S5) of adjusting a working condition for a work on the basis of the psychological state acquired in the acquisition step; and
a presentation step (S10) of presenting the operator with a working condition adjusted in the adjustment step.

8. A non-transitory storage medium recording a work assistance program causing a computer to execute:
an acquisition process of acquiring a psychological state of an operator who performs a predetermined work;
an adjustment process of adjusting a working condition for the work on the basis of the psychological state acquired in the acquisition process; and
a presentation process of causing a presentation part for presenting the working condition to the operator to present a working condition adjusted in the adjustment process.
